(19) Europäisches Patentamt
European Patent Office
Office européen des brevets

(11) Veröffentlichungsnummer : **0 088 323**
**B1**

(12) # EUROPÄISCHE PATENTSCHRIFT

(45) Veröffentlichungstag der Patentschrift : **06.05.87**

(21) Anmeldenummer : **83101929.4**

(22) Anmeldetag : **28.02.83**

(51) Int. Cl.[4] : **C 07 D513/04**, A 61 K 31/41, A 61 K 31/445, A 61 K 31/535, A 61 K 31/55 // (C07D513/04, 285:00, 235:00)

(54) **Imidazothiadiazolalkencarbonsäureamide, neue Zwischenprodukte zu ihrer Herstellung, ihre Herstellung und ihre Verwendung in Arzneimitteln.**

(30) Priorität : **09.03.82 DE 3208437**

(43) Veröffentlichungstag der Anmeldung : **14.09.83 Patentblatt 83/37**

(45) Bekanntmachung des Hinweises auf die Patenterteilung : **06.05.87 Patentblatt 87/19**

(84) Benannte Vertragsstaaten : **AT BE CH DE FR GB IT LI LU NL SE**

(56) Entgegenhaltungen :
**EP-A- 0 005 783**
**EP-A- 0 041 215**
**JOURNAL OF MEDICINAL CHEMISTRY, Band 23, 1980, Washington I.T. BURNISH et al. "Cerebrovasodilatation through selective inhibition of the enzyme carbonic anhydrase. 2. Imidazo[2,1-b]thiadiazole and imidazo[2,1-b]thiazolesulfonamides", Seiten 117-121**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Ingendoh, Axel, Dr.**
**Unterste Dillenberg 18**
**D-5620 Velbert 15 (DE)**
Erfinder : **Meyer, Horst, Dr.**
**Henselweg 11**
**D-5600 Wuppertal 1 (DE)**
Erfinder : **Garthoff, Bernward, Dr.**
**Händelstrasse 22**
**D-4010 Hilden (DE)**

**EP 0 088 323 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Imidazothiadiazolalkencarbonsäureamide, diese Verbindungen zur Anwendung bei der therapeutischen Behandlung des menschlichen und tierischen Körpers, verschiedene Verfahren zu ihrer Herstellung und neue Zwischenprodukte, die bei ihrer Herstellung eingesetzt werden ; ferner Arzneimittel, insbesondere in Antihypertensiva, Diuretika und Uricosurica, die diese neuen Verbindungen enthalten.

Es ist bereits bekannt geworden, daß bestimmte Imidazothiadiazole biologische Wirkungen, insbesondere antithrombotische und antimikrobielle Eigenschaften besitzen (vgl. DE-OS 30 20 421).

Weiterhin ist bekannt, daß bestimmte Imidazothiadiazole und Imidazothiazolsulfonamide cerebrale Wirkungen besitzen (J. med. Chem., *1980,* 117, IC. Barnisch et al.).

Die antihypertensive, diuretische und uricosurische Wirkung von anderen Imidazoalkencarbonsäureamiden wurde ebenfalls bereits erwähnt (DE-OS 30 43 158).

Die Erfindung betrifft neue Imidazolthiadiazolalkencarbonsäureamide der allgemeinen Formel (I)

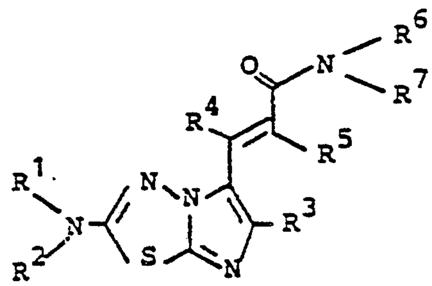

(I)

in welcher

$R^1$ für Wasserstoff, Phenyl oder einen geradkettigen verzweigten oder cyclischen gesättigten oder ungesättigten Kohlenwasserstoffrest mit bis zu 6 Kohlenstoffatomen steht, der gegebenenfalls einmal durch Sauerstoff, NH, N-Alkyl mit 1 bis 4 C-Atomen oder N-Benzyl unterbrochen ist und der gegebenenfalls substituiert ist durch Halogen,

$R^2$ entweder für den Rest $CXR^8$ steht, wobei $R^8$ die für $R^1$ angegebene Bedeutung hat (gleich oder verschieden) und wobei X für Sauerstoff oder den Rest $NR^9R^{10}$ steht, wobei $R^9$ und $R^{10}$ gleich oder verschieden sind und jeweils die für $R^1$ angegebene Bedeutung besitzen oder

$R^1$ und $R^2$ gemeinsam für eine Gruppe der allgemeinen Formel $= C(R^{12})(Y\text{-}R^{13})$ stehen, wobei Y für den Rest N-Alkyl mit 1 bis 4 C-Atomen steht und $R^{12}$ und $R^{13}$ gleich oder verschieden sind und jeweils die für $R^1$ angegebene Bedeutung besitzen und mit $R^1$ gleich oder verschieden sind,

$R^3$ die für $R^1$ angegebene Bedeutung besitzt und mit $R^1$ gleich oder verschieden ist oder für Phenyl, Furyl, Thienyl, Pyrimidyl oder Pyridyl steht, wobei die Ringe gegebenenfalls substituiert sind durch ein oder zwei gleiche oder verschiedene Substituenten aus der Gruppe Halogen, Nitro, Trifluormethyl, Phenyl, Alkyl, Alkoxy, Dialkylamino oder $SO_n$-Alkyl (n = 0 oder 2), wobei die genannten Alkyl- oder Alkoxy-Reste 1 bis 4 Kohlenstoffatome enthalten,

$R^4$ und $R^5$ gleich oder verschieden sind und für Wasserstoff oder Alkyl mit 1 bis 4 Kohlenstoffatomen stehen und

$R^6$ unf $R^7$ die für $R^1$ angegebene Bedeutung besitzen und mit $R^1$ gleich oder verschieden sind oder gemeinsam mit dem Stickstoffatom einen 5- bis 7-gliedrigen Ring bilden, der gegebenenfalls durch ein Sauerstoff- oder Stickstoffatom unterbrochen ist, wobei der Stickstoff gegebenenfalls durch Wasserstoff, Alkyl mit 1 bis 4 Kohlenstoffatomen oder Benzyl substituiert ist und wobei dieser 5- bis 7-gliedrige Ring gegebenenfalls durch 1 oder 2 gleiche oder verschiedene Substituenten aus der Gruppe Halogen, Benzyl, Trifluormethyl, Alkyl oder Alkoxy mit jeweils 1 bis 4 Kohlenstoffatomen substituiert ist oder wobei dieser Ring gegebenenfalls in einem aromatischen Ring mit 6 oder 10 C-Atomen kondensiert sein kann,

sowie ihre stereoisomeren Formen der verschiedenen Enantiomeren, Diastereomeren und E/Z-Isomeren sowie ihre pharmazeutisch unbedenklichen Säureadditionssalze.

Die Herstellung der erfindungsgemäßen Imidazothiadiazolalkencarbonsäureamide der allgemeinen Formel (I) erfolgt, indem man

a) Carbonylverbindungen der allgemeinen Formel (II)

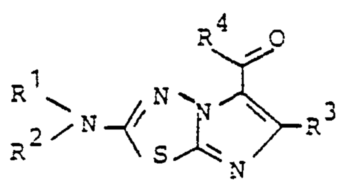

(II)

in welcher $R^1$ bis $R^4$ die oben angegebene Bedeutung haben, mit Phosphonatverbindungen der allgemeinen Formel (III)

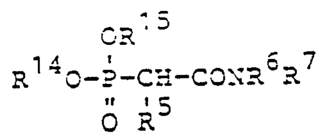

(III)

in welcher

$R^5$ bis $R^7$ die oben angegebene Bedeutung haben, und

$R^{14}$ und $R^{15}$ für gegebenenfalls substituiertes Alkyl oder Aralkyl stehen,

in Gegenwart von starken Basen und in inerten organischen Lösungsmitteln bei Temperaturen zwischen — 20 und 110 °C umsetzt, wobei, im Falle $R^8 = CH_3$ und X = 0, Alkencarbonsäureamide der allgemeinen Formel (I) mit $R^2$ = H erhalten werden oder

b) Alkencarbonsäureamide der allgemeinen Formel (I), in der $R^1$ bis $R^7$ die oben angegebene Bedeutung haben und $R^2$ = Wasserstoff ist, mit geeigneten Acylierungsmitteln der allgemeinen Formel (IV)

$$R^{2'}—Cl \qquad (IV)$$

in welcher $R^{2'}$ die oben für $R^2$ angegebene Bedeutung besitzt, aber nicht Wasserstoff bedeutet, in inerten organischen Lösungsmitteln, gegebenenfalls in Gegenwart einer organischen Base, umsetzt, oder

c) Carbonylverbindungen der allgemeinen Formel (II) mit Acetamidderivaten der allgemeinen Formel (V)

$$R^5—CH_2—CONR^6R^7 \qquad (V)$$

in welcher $R^5$, $R^6$ und $R^7$ die oben angegebene Bedeutung haben, in Gegenwart von sauren oder basischen Katalysatoren und gegebenenfalls in Gegenwart von inerten organischen Lösungsmitteln bei Temperaturen zwischen 20 und 200 °C umsetzt, oder

d) Alkencarbonsäuren der allgemeinen Formel (VI)

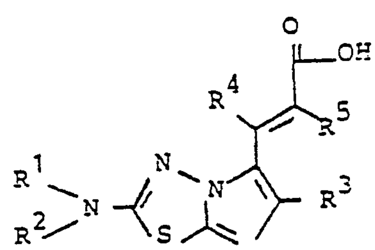

(VI)

in welcher $R^1$ bis $R^5$ die oben angegebene Bedeutung haben, mit Aminen der allgemeinen Formel (VII)

$$HNR^6R^7 \qquad (VII)$$

in welcher $R^6$ und $R^7$ die oben angegebene Bedeutung haben, gegebenenfalls nach Aktivierung der Carboxylgruppe über das entsprechende Säurechlorid z. B. (durch Thionylchlorid) in üblicher Weise in organischen inerten Lösungsmitteln bei Temperaturen zwischen 20 und 150 °C amidiert.

Je nach Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemate und die Diastereomerengemische sind Gegenstand der vorliegenden Erfindung. Die Racemate lassen sich ebenso wie die Diastereomeren in bekannter Weise in die reinen Stereoisomeren trennen (vgl. E. L. Eliel, Stereochemistry of Carbon Compounds, McGraw Hill 1962).

Darüber hinaus werden die durch die Doppelbindung der Seitenkette möglichen E/Z-Isomeren beansprucht, die nach bekannten Verfahren hergestellt oder nach bekannten Verfahren ineinander überführt werden können. Als bekanntes Verfahren sei z. B. die Bestrahlung mit UV-Strahlen genannt.

Die als Ausgangsverbindungen einsetzbaren Carbonylverbindungen der allgemeinen Formel (II) sind

bisher nicht bekannt, können aber in analoger Weise nach bekannten Methoden aus Imidazothiadiazolen der allgemeinen Formel (VIII)

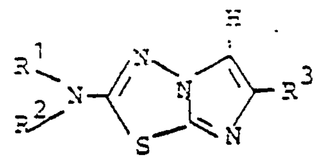

(VIII)

in welcher $R^1$ bis $R^3$ die oben angegebene Bedeutung haben, indem man sie mit Dimethylformamid in Gegenwart von Phosphoroxychlorid bei Temperaturen von 20 °C-170 °C umsetzt, hergestellt werden, (vgl. z. B. L. Pentimalli et al., Boll. Sci. Fac. Chim. Ind. Bologna *23*, 181 (1965) ; C. A. *63*, 17848 e (1965), D. Bower et al., J. Chem. Soc. 1955, 2834, A. Hetzheim et al., Chem. Ber. *103*, 3533 (1970), H. Beyer et al., Z. Chem *2*, 152 (1962) und S. Kano, Yagukagu Zasski *92*, 935 (1972)).

Die als Ausgangsverbindungen einsetzbaren Alkencarbonsäuren der allgemeinen Formel (VI) sind ebenfalls neu. Sie können nach bekannten Methoden hergestellt werden, indem man

a) Carbonylverbindungen der allgemeinen Formel (II), in welcher $R^1$ bis $R^4$ die oben angegebene Bedeutung haben, mit Phosphonatverbindungen der allgemeinen Formel (IX)

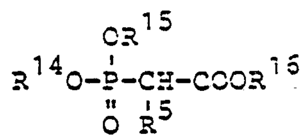

(IX)

in welcher

$R^5$, $R^{14}$ und $R^{15}$ die oben angegebene Bedeutung haben und

$R^{16}$ für H, Trialkyl- oder Triarylsilyl oder für einen Rest der gleichen Bedeutung wie $R^{14}$ steht,

in Gegenwart von starken Basen in inerten organischen Lösungsmitteln zu den Alkencarbonsäurederivaten umsetzt und diese dann mit Säuren oder Laugen zu den freien Carbonsäuren verseift (vgl. W.S. Wadsworth et al., JACS *83*, 1733 (1961)), oder

b) Carbonylverbindungen der allgemeinen Formel (II), in welcher $R^1$ bis $R^3$ die oben angegebene Bedeutung haben, $R^4$ gleich Wasserstoff ist (Aldehyde), mit Malonsäuren der allgemeinen Formel $R^5—CH(COOH)_2$ in welcher $R^5$ die oben angegebene Bedeutung hat, oder mit Meldrumsäure in Gegenwart von inerten organischen Lösungsmitteln gegebenenfalls in Gegenwart von Kondensationsmitteln umsetzt (vgl. G. Jones, Org. Reactions, Vol. 15, S. 204 ff).

Die als Ausgangsverbindungen einsetzbaren Imidazothiadiazole der allgemeinen Formel (VIII) sind bekannt oder können z. B. durch Umsetzung von 2-Brom-5-amino-1,3,4-thiadiazol mit α-Halogenketonen der allgemeinen Formel $Y—CH_2—CO—R^3$, wobei $R^3$ die oben angegebene Bedeutung besitzt, und Y für Cl, Br oder J steht in organischen Lösungsmitteln und anschließender Behandlung mit einem Überschuß von Aminen der allgemeinen Formel $H_2N—R^1$, gefolgt von einer Umsetzung mit Acylierungsmitteln der allgemeinen Formel $R^8CXCl$, wobei $R^1$, $R^8$ und X die oben angegebene Bedeutung besitzen, erhalten werden.

Falls nicht ausdrücklich anders angegeben steht in der vorliegenden Anmeldung Aryl für einen aromatischen Kohlenwasserstoffrest mit 6 bis 14 C-Atomen, insbesondere für Phenyl oder Naphthyl.

Besonders hervorgehoben seien Verbindungen der allgemeinen Formel (I), in welcher

$R^1$ für Wasserstoff, Phenyl oder einen geradkettigen verzweigten oder cyclischen gesättigten oder ungesättigten Kohlenwasserstoffrest mit bis zu 6 Kohlenstoffatomen steht, der gegebenenfalls einmal durch Sauerstoff, NH, N-Alkyl mit 1-4 C-Atomen oder N-Benzyl unterbrochen ist und der gegebenenfalls substituiert ist durch Halogen,

$R^2$ für Wasserstoff oder den Rest $CXR^8$ steht, wobei $R^8$ die für $R^1$ angegebene Bedeutung hat (gleich oder verschieden) und wobei X für Sauerstoff oder den Rest $NR^9R^{10}$ steht, wobei $R^9$ und $R^{10}$ gleich oder verschieden sind und jeweils die für $R^1$ angegebene Bedeutung besitzen oder

$R^1$ und $R^2$ gemeinsam für eine Gruppe der allgemeinen Formel $= C(R^{12})(Y-R^{13})$ stehen, wobei Y für den Rest N-Alkyl mit 1 bis 4 C-Atomen steht und $R^{12}$ und $R^{13}$ gleich oder verschieden sind und jeweils die für $R^1$ angegebene Bedeutung besitzen und mit $R^1$ gleich oder verschieden sind,

$R^3$ die für $R^1$ angegebene Bedeutung besitzt und mit $R^1$ gleich oder verschieden ist oder für Phenyl, Furyl, Thienyl, Pyrimidyl oder Pyridyl steht, wobei die Ringe gegebenenfalls substituiert sind durch ein oder zwei gleiche oder verschiedene Substituenten aus der Gruppe Halogen, Nitro, Trifluormethyl, Phenyl, Alkyl, Alkoxy, Dialkylamino oder $SO_n$-Alkyl ($n = 0$ oder 2), wobei die genannten Alkyl- und Alkoxyreste jeweils 1 bis 4 Kohlenstoffatomen enthalten.

$R^4$ und $R^5$ gleich oder verschieden sind und für Wasserstoff oder Alkyl mit 1-4 Kohlenstoffatomen stehen und

$R^6$ und $R^7$ die für $R^1$ angegebene Bedeutung besitzen und mit $R^1$ gleich oder verschieden sind oder gemeinsam mit dem Stickstoffatom einen 5- bis 7-gliedrigen Ring bilden, der gegebenenfalls durch ein Sauerstoff- oder Stickstoffatom unterbrochen ist, wobei der Stickstoff gegebenenfalls durch Wasserstoff, Alkyl mit 1-4 Kohlenstoffatomen oder Benzyl substituiert ist und wobei dieser 5- bis 7-gliedrige Ring gegebenenfalls durch 1 oder 2 gleiche oder verschiedene Substituenten aus der Gruppe Halogen, Benzyl, Trifluormethyl, Alkyl oder Alkoxy mit jeweils 1-4 Kohlenstoffatomen substituiert ist oder wobei dieser Ring gegebenenfalls mit einem aromatischen Ring mit 6 oder 10 C-Atomen kondensiert sein kann, sowie ihre stereoisomeren Formen der verschiedenen Enantiomeren, Diastereomeren und Z/E-Isomeren sowie ihre pharmazeutisch unbedenklichen Säureadditionssalze.

Gegenstand der vorliegenden Erfindung sind auch die als Zwischenprodukte verwendbaren Carbonylverbindungen der allgemeinen Formel (II) und die Alkencarbonsäuren der allgemeinen Formel (VI) sowie Verfahren zu ihrer Herstellung.

Die neuen erfindungsgemäßen Verbindungen zeichnen sich überraschenderweise durch starke biologische Wirkungen aus. Insbesondere besitzen sie ausgeprägte diuretische und saluretische Wirkungen und können daher als Diuretika, Saluretika und Antihypertensiva verwendet werden. Bei Tierverscuhen an Mäusen, Ratten bzw. Hunden zeigt sich, daß die erfindungsgemäßen Verbindungen bei oraler Applikation bereits bei Dosierungen unter 10 mg/kg eine ausgeprägte diuretische und saluretische Wirkung bei gleichzeitiger guter Verträglichkeit besitzen. Bei Kenntnis des Standes der Technik konnten diese vorteilhaften Eigenschaften nicht erwartet werden.

Die überraschenden und vorteilhaften Wirkungen der erfindungsgemäßen Verbindungen lassen sich nach den folgenden Testmethoden ermitteln :

A) Antihypertensive Wirkung an Ratten

Die Blutdruckwirkung wird ermittelt an Goldblatthochdruckratten nach Breuninger, H. : Methoden zur unblutigen Messung des Blutdruckes an Kleintieren, Arzneimittelforsch. *6,* 222-225 (1965).

B) Diuretische Wirkung an Ratten

Nüchterne männliche Ratten im Gewicht von 150 bis 250 g (SPF, Wistar, je n = 4 Paare) werden mit 10 ml/kg p. o. Tylose-Suspension (0,5 %) als Kontrollen bzw. mit 100 mg/kg p. o. Prüfsubstanz in 10 ml/kg p. o. Tylose-Suspension mittels Schlundsonde behandelt. Die Tiere werden in Stoffwechselkäfige verbracht und die Harn- und Elektrolytausscheidung über 6 Stunden bestimmt ($Na^+$ und $K^+$ Bestimmung : IL-Flammenphotometer).

C) Diuretische Wirkung an Hunden

Bei nüchternen, wachen weiblichen Beagle-Hunden wird die Harnblase katheterisiert und die Harn- und Elektrolytausscheidung über 180 Minuten (fraktioniert über jeweils 30 Minuten) bestimmt. Die Tiere erhalten während dieser Zeit über eine Dauerinfusion eine Elektrolytlösung intravenös und am Beginn des Versuches die Prüfsubstanz in 1 ml/kg Tylose-Suspension (0,5 %) oral zugeführt. Der Harn wird auf $Na^+$, $K^+$, Chlor, Bicarbonat und pH analysiert.

D) Diuretische Wirkung an Mäusen

Nüchterne männliche SPF-Mäuse im Gewicht von 20 bis 25 g (n = 6 × 3 Tiere/Käfig) erhalten 100 ml/kg Tylose-Suspension (0,5 %) als Kontrollen, bzw. 100 mg/kg Prüfsubstanz in Tylose-Suspension oral zugeführt.

Die Ausscheidung von Harn, $Na^+$ und $K^+$ bzw. Harnsäure wird über 2 und 4 Stunden in Stoffwechselkäfigen bestimmt.

E) Phenolrot-Retentionstest an Ratten

Zum Nachweis von uricosurischer Wirksamkeit wird an wachen, nüchternen männlichen Ratten (SPF-Wistar, Gewicht 180 bis 250 g) der Einfluß von erfindungsgemäßen Verbindungen auf den Phenolrot-Blutspiegel dargestellt. Nach der Methode von Kreppel, E. (Med. exp. 1 (1959), 285-289) erhalten je 8 Tiere 75 mg/kg Phenolrot in 5 ml/kg Kochsalzlösung intraperitoneal, nachdem ihnen 30 Minuten vorher entweder 10 mg/kg Tylose-Suspension (0,5 %) als Kontrollen oder 100 mg/kg Prüfsubstanz in Tylose-Suspension verabreicht worden war. Plasma wird durch Punktion des retroorbitalen Venenplexus 30. 60 und 120 Minuten nach Gabe von Phenolrot, bzw. 60, 90 und 150 Minuten nach Substanzgabe gewonnen, mit NaOH versetzt und die Extinktion bei 546 nm im Photometer (Eppendorf) bestimmt.

Eine potentielle uricosurische Wirksamkeit liegt vor, wenn die Extinktionswerte signifikant höher als in der Kontrollgruppe sind.

Die neuen erfindungsgemäßen Verbindungen sind als Arzneimittel verwendbare Substanzen. Sie bewirken bei oraler oder parenteraler Anwendung eine Steigerung der Wasser- und Salzausscheidung und können daher zur Behandlung œdematöser und hypertoner Zustände und zur Ausschwemmung toxischer Substanzen dienen.

Darüber hinaus können die Verbindungen bei akutem Nierenversagen eingesetzt werden. Insbesondere zeigen sie auch eine vorteilhafte uricosurische Wirkung.

Die neuen Verbindungen können in bekannter Weise in die üblichen Formulierungen übergeführt werden, wie Tabletten, Kapseln, Dragees, Pillen, Granulate, Sirupe, Emulsionen, Suspensionen und Lösungen, unter Verwendung inerter, nichttoxischer, pharmazeutisch geeigneter Trägersubstanzen oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreiche.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt :

Wasser, nichttoxische organische Lösungsmittel wie Paraffine (z. B. Erdölfraktionen), pflanzliche Öle (z. B. Erdnuß-Sesamöl), Alkohole (z. B. Ethylalkohol, Glycerin), Glykole (z. B. Propylenglykol, Polyethylenglykol), feste Trägerstoffe wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzucker), Emulgiermittel wie nichtionogene und anionische Emulgatoren (z. B. Polyoxyethylen-Fettsäure-Ester, Polyoxyethylen-Fettalkohol-Ether, Alkylsulfonate und Arylsulfonate), Dispergiermittel (z. B. Lignin, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral.

Im Falle der oralen Anwendung können Tabletten selbstverständlich außer den genannten Trägerstoffen auch Zusätze wie Natriumcitrat, Calciumcarbonat und Calciumphosphat zusammen mit verschiedenen Zuschlagstoffen wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den oben genannten Hilfsstoffen mit verschiedenen Geschmacksaufbessern oder Farbstoffen versetzt werden.

Für den Fall der parenteralen Anwendung können Lösungen der Wirkstoffe unter Verwendung geeigneter flüssiger Trägermaterialien eingesetzt werden. Als besonders vorteilhaft für den Fall der parenteralen Anwendung hat sich die Tatsache herausgestellt, daß die erfindungsgemäßen Verbindungen in Wasser gut lösliche Salze zu bilden vermögen. Diese Salze werden erhalten, wenn man die erfindungsgemäßen Verbindungen in einem geeigneten Lösungsmittel mit der äquimolaren Menge einer nichttoxischen anorganischen oder organischen Base vereinigt. Als Beispiele seien genannt : Natronlauge, Kalilauge, Ethanolamin, Diethanolamin, Triethanolamin, Amino-tris-hydroxymethyl-methan, Glucosamin, N-Methylglucosamin. Derartige Salze können auch für die orale Anwendung der erfindungsgemäßen Verbindungen eine erhöhte Bedeutung besitzen, indem sie die Resorption je nach Wunsch beschleunigen oder verzögern. Als Beispiele seien außer den oben bereits erwähnten Salzen genannt : Magnesiumsalze, Calciumsalze, Aluminiumsalze und Eisensalze.

Im allgemeinen hat es sich als vorteilhaft erwiesen, bei parenteraler Applikation Mengen von etwa 0,05 bis 100 mg/kg, vorzugsweise etwa 0,1 bis 10 mg/kg Körpergewicht pro Tag zur Erzielung wirksamer Ergebnisse zu verabreichen und bei oraler Applikation beträgt die Dosierung etwa 0,1 bis 500 mg/kg, vorzugsweise 0,5 bis 100 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in wenigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen.

Diese Angaben gelten sowohl für die Anwendung der erfindungsgemäßen Verbindungen in der Veterinär- als auch in der Humanmedizin.

Die bei der Durchführung des erfindungsgemäßen Herstellungsverfahrens eingesetzten Phosphonatverbindungen der allgemeinen Formel (III) sind bekannt oder können nach bekannten Methoden hergestellt werden (vgl. I. Shahak et al. Isr. J. Chem. *7*, 585 (1969)).

Als starke Basen zur Verwendung bei der Durchführung der Verfahrensvariante a) seien beispielhaft genannt :

Alkalihydride, wie Natriumhydrid, Kaliumhydrid, Lithiumhydrid und Alkalialkoholate wie Natriumethylat, Kaliumethylat oder Kaliummethylat oder Alkalimetallalkyle wie Methyllithium oder Butyllithium.

Die bei der Durchführung der Verfahrensvariante c) eingesetzten Acetamidderivate der allgemeinen Formel (V) sind bekannt oder können nach bekannten Methoden hergestellt werden (vgl. a) Brit. Pat. 715 896 (1954) ; C.A. *49*, 13290d (1955) ; b) DBP 1 142 859 (1960) ; C.A. *59*, 7377c (1963)).

Bei dieser Verfahrensvariante c) werden vorzugsweise saure oder basische Katalysatoren eingesetzt, von denen beispielhaft genannt seien ; basische Amine wie Dialkylamine, Piperidin oder Pyridin oder anorganische Säuren, insbesondere Salzsäure oder Kondensationsmittel wie Carbonsäureanhydride.

Die gemäß Verfahrensvariante d) eingesetzten Alkensäuren der allgemeinen Formel (VI) sind bisher noch nicht bekannt geworden, lassen sich aber nach den oben angegebenen Verfahren in an sich bekannter Weise herstellen. Die für die Umsetzung mit Aminen zweckmäßige Aktivierung der freien Carboxylgruppe erfolgt vorzugsweise über das entsprechende Säurehalogenid, insbesondere über das entsprechende Säurechlorid unter Verwendung von Halogenidbildnern, wie z. B. Thionylchlorid, Phosphortrichlorid und Phosphorpentachlorid.

Bei allen erfindungsgemäßen Verfahren können als Verdünnungsmittel die üblichen inerten organischen Lösungsmittel eingesetzt werden. Hierzu gehören vorzugsweise Ether wie Diethylether, Glykolether wie Glykoldimethylether, Dioxan, Tetrahydrofuran oder Alkohole wie Methanol, Ethanol, Propanol, Butanol, Benzylalkohol oder Sulfoxide wie Dimethylsulfoxid, Basen wie Pyridin, Chinolin, Picolin oder Kohlenwasserstoffe wie Benzol, Toluol, Xylol sowie Dimethylformamid.

Bei der Herstellung der Alkensäuren der allgemeinen Formel (VI) werden für die Verseifung der korrespondierenden Ester als Basen vorzugsweise benutzt : Alkalimetallhydroxide wie Natriumhydroxid, Kaliumhydroxid, Erdalkalihydroxid wir Bariumhydroxid oder Calciumhydroxid.

Bei der Herstellung über die Aldehyde der allgemeinen Formel (II) mit $R^4$ gleich Wasserstoff mit Malonsäuren der allgemeinen Formel $R^5$—$CH(COOH)_2$ werden als Kondensationsmittel vorzugsweise verwendet : Pyridin, substituierte Pyridinderivate wie Dialkylaminopyridine, Chinolin, Isochinolin, Dialkylamine wie Dimethylamin, Dibutylamin, Pyrrolidin, Piperidin und ähnliche stickstoffhaltige organische Basen.

Die nachfolgenden Beispiele A bis G erläutern die Herstellung der erfindungsgemäß als Ausgangssubstanzen verwendeten Imidazothiadiazole der allgemeinen Formel (VIII) (Tabelle 1).

Die Beispiele H bis L erläutern die Herstellung der erfindungsgemäß verwendeten Carbonylverbindungen der allgemeinen Formel (II) (Tabelle 2).

Die dann folgenden Beispiele erläutern die Herstellung der erfindungsgemäßen Imidazothiadiazolalkensäureamide der allgemeinen Formel (I) (Tabelle 3).

## Ausführungsbeispiele

Beispiele für die Herstellung von Imidazothiadiazolen der allgemeinen Formel VIII

### Beispiel A

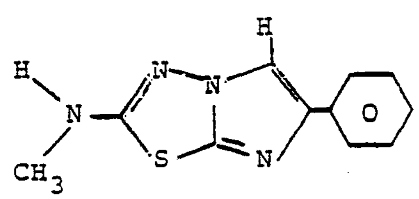

2-Methylamino-6-phenylimidazo[2,1-b]-1,3,4-thiadiazol

18,0 g 2-Brom-5-amino-1,3,4-thiadiazol (0,1 Mol) und 20,0 g ω-Bromacetophenon (0,1 Mol) werden in 250 ml Dimethylformamid 4,5 Stunden auf 100 °C erwärmt. Nach dem Abkühlen auf Raumtemperatur wird mit 100 ml Eiswasser versetzt und der Niederschlag abgesaugt. Nach dem Trocknen wird in Chloroform aufgenommen und nach Zusatz von Ether umkristallisiert. Das rohe 2-Brom-6-phenylimidazo[2,1-b]-1,3,4-thiadiazol wird anschließend in 250 ml Ethanol mit 25 ml 30 %iger Methylaminlösung zwei Stunden zum Rückfluß erwärmt. Nach dem Abkühlen fällt man durch Zusatz von 200 ml Wasser aus, saugt den Niederschlag ab und kristallisiert aus Chloroform/Ether um.

Ausbeute : 12,6 g (55 %) Fp. : 177 °C.

(Siehe Beispiel B Seite 8 f.)

Beispiel B

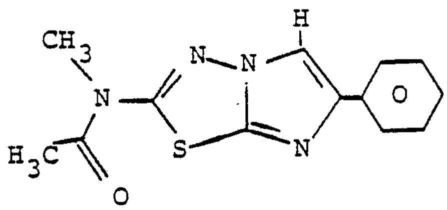

N-Acetyl-N-methyl-2-amino-6-phenylimidazo[2,1-b]-1,3,4-thiadiazol

23,0 g 2-Methylamino-6-phenyl-imidazo-[2,1-b]-1,3,4-thiadiazol (0,1 Mol) werden in 200 ml Acetanhydrid zum Rückfluß erhitzt. Nach dem Abkühlen fügt man 200 ml Ether zu, saugt den Niederschlag ab und trocknet.

Ausbeute : 25,8 g (99 %) Fp. : 280 °C.

Tabelle 1

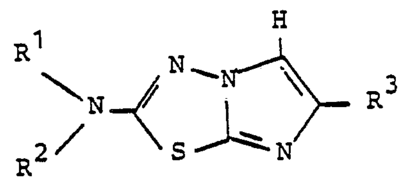

(VIII)

| Beispiel | $R^1$ | $R^2$ | $R^3$ | Fp.: | Ausbeute % d.Th. |
|---|---|---|---|---|---|
| C | H | $nC_3H_7$ | $C_6H_5$ | 174°C | 87 |
| D | $CH_3CO$ | $nC_3H_7$ | $C_6H_5$ | 160°C | 73 |
| E | H | $C_2H_5$ | $C_6H_5$ | 162°C | 80 |
| F | $CH_3CO$ | $C_2H_5$ | $C_6H_5$ | 215°C | 60 |
| G | H | H | $C_6H_5$ | 192°C | 90 |

Beispiele für die Herstellung von Carbonylverbindungen der allgemeinen Formel II

Beispiel H

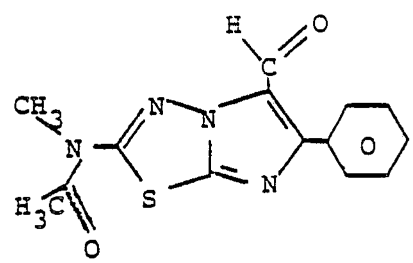

N-Acetyl-N-methyl-2-amino-6-phenyl-imidazo-[2,1-b]-1,3,4-thiadiazol-5-carbaldehyd

27,2 g N-Acetyl-N-methyl-2-amino-6-phenyl-imidazo-[2,1-b]-1,3,4-thiadiazol (0,1 Mol) werden bei Raumtemperatur zu einer kalt hergestellten Lösung von 18 ml Phosphoroxychlorid in 300 ml trockenem Dimethylformamid gegeben und 45 Minuten auf 80 °C erwärmt. Nach dem Abkühlen fügt man 250 ml Eiswasser zu, saugt ab und löst den Niederschlag in Chloroform. Nach dem Trocknen über Natriumsulfat wird eingeengt und nach Zusatz von Ether umkristallisiert.

Ausbeute : 27,0 g (90 %) Fp. : 237 °C

Tabelle 2

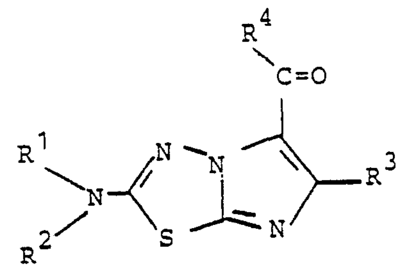

| Beispiel | $R^1$ | $R^2$ | $R^3$ | $R^4$ | Ausbeute % | Fp. |
|---|---|---|---|---|---|---|
| I | $CH_3CO$ | $C_2H_5$ | $C_6H_5$ | H | 91 | 181°C |
| K | $CH_3CO$ | $nC_3H_7$ | $C_6H_5$ | H | 95 | 183–185°C |
| L | 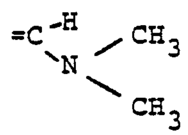 | | $C_6H_5$ | H | 69 | 159°C |

Beispiele für die Herstellung von Imidazothiadiazolalkencarbonsäureamiden der allgemeinen Formel (I)

Beispiel 1

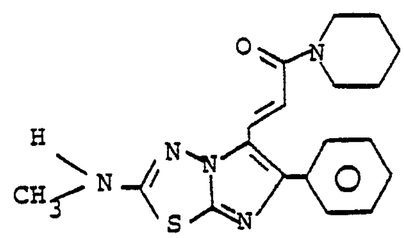

N-[β-(2-Methylamino-6-phenylimidazo[2,1-b]-1,3,4-thiadiazol-5-yl)-E-propenoyl]piperidin

Zu 0,33 g Natriumhydrid 80 % (0,011 Mol) (mit Petrolether entölt) in 100 ml Toluol (wasserfrei) fügt man 2,9 g Diethylphosphonoessigsäurepiperidid (0,011 Mol) und erwärmt auf 60 °C, bis die Wasserstoffentwicklung beendet ist. Bei Raumtemperatur gibt man 2,6 g N-Acetyl-N-methyl-2-amino-6-phenylimidazo-[2,1-b]-1,3,4-thiadiazol-5-carbaldehyd hinzu und rührt eine Stunde bei 50 °C. Nach dem Abkühlen versetzt man mit 20 ml Ethanol und nach 30 Minuten mit 100 ml Wasser. Die organische Phase wird abgetrennt, die wäßrige Phase noch zweimal mit Chloroform extrahiert und die organischen Phasen über Natriumsulfat getrocknet. Nach dem Einengen kristallisiert man aus Chloroform/Ether um.

Ausbeute : 2,49 g (68 %) Fp. : 264 °C.

Beispiel 2

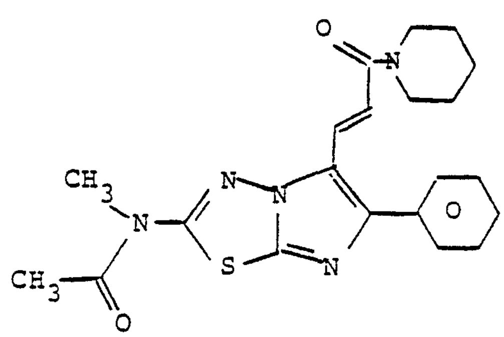

N-[β-(N-Acetyl-N-methyl-2-amino-5-phenyl-imidazo-[2,1-b]-1,3,4-thiadiazol-5-yl)-E-propenoyl]-piperidin

3,67 g N-β-(2-Methylamino-6-phenyl-imidazo-[2,1-b]-1,3,4-thiadiazol-5-yl)-E-propenoyl-piperidin (0,01 Mol) werden in 50 ml Acetanhydrid eine Stunde zum Rückfluß erwärmt. Nach dem Abkühlen versetzt man mit 50 ml Ether und saugt den Niederschlag ab.

Ausbeute : 2,94 g (72 %) Fp. : 209 °C.

Tabelle 3

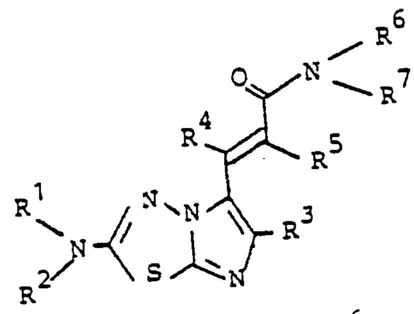

| Beispiel Nr. | $R^1$ | $R^2$ | $R^3$ | $R^4$ | $R^5$ | 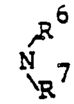 | Ausbeute % | Fp. |
|---|---|---|---|---|---|---|---|---|
| 3 | H | $CH_3$ | $C_6H_5$ | H | H | 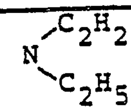 | 60 | 236-40°C |
| 4 | H | $CH_3$ | $C_6H_5$ | H | H | 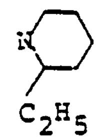 | 30 | 204°C |
| 5 | $CH_3NH{-}CO$ | $CH_3$ | $C_6H_5$ | H | H | 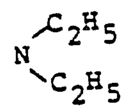 | 60 | 161°C |
| 6 | H | $nC_3H_7$ | $C_6H_5$ | H | H | 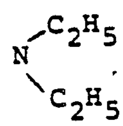 | 60 | 185°C |
| 7 | $CH_3CO$ | $nC_3H_7$ | $C_6H_5$ | H | H | 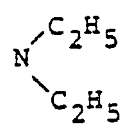 | 73 | 164°C |
| 8 | H | $nC_3H_7$ | $C_6H_5$ | H | H | 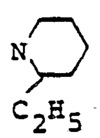 | 50 | 222°C |
| 9 | H | $nC_3H_7$ | $C_6H_5$ | H | H | 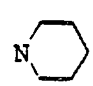 | 70 | 262°C |
| 10 | $CH_3CO$ | $nC_3H_7$ | $C_6H_5$ | H | H | 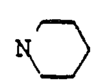 | 20 | 219°C |
| 11 | H | $nC_3H_7$ | $C_6H_5$ | H | H | 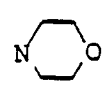 | 50 | 278°C |
| 12 | $CH_3CO$ | $nC_3H_7$ | $C_6H_5$ | H | H | 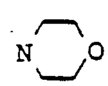 | 60 | 205°C |

Tabelle 3 (Fortsetzung)

| Beispiel Nr. | $R^1$ | $R^2$ | $R^3$ | $R^4$ | $R^5$ | 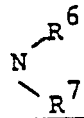 | Ausbeute % | Fp. |
|---|---|---|---|---|---|---|---|---|
| 13 | H | $C_2H_5$ | $C_6H_5$ | H | H | 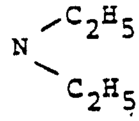 | 66 | 167°C |
| 14 | H | $C_2H_5$ | $C_6H_5$ | H | H | 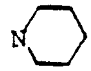 | 71 | 232°C |
| 15 | H | $C_2H_5$ | $C_6H_5$ | H | H | 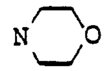 | 68 | 216°C |
| 16 | $CH_3CO$ | $CH_3$ | $C_6H_5$ | H | H | 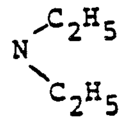 | 64 | 209°C |
| 17 | $C_6H_5$-CO | $CH_3$ | $C_6H_5$ | H | H | 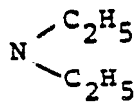 | 69 | 257°C |
| 18 | $C_2H_5$-CO | $CH_3$ | $C_6H_5$ | H | H | 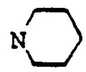 | 73 | 214°C |
| 19 | $C_2H_5$-CO | $CH_3$ | $C_6H_5$ | H | H | 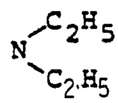 | 80 | 170°C |
| 20 | $CH_3NH$-CO | $CH_3$ | $C_6H_5$ | H | H | 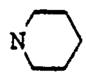 | 10 | 177°C |
| 21 | $(CH_3)_2N$-CO | $CH_3$ | $C_6H_5$ | H | H | 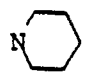 | 95 | 160°C |
| 22 | $CH_3CO$ | $C_2H_5$ | $C_6H_5$ | H | H | 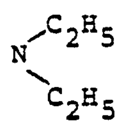 | 75 | 160°C |
| 23 | $C_2H_5CO$ | $C_2H_5$ | $C_6H_5$ | H | H | 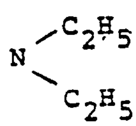 | 76 | 187°C |
| 24 | $C_6H_5$-CO | $C_2H_5$ | $C_6H_5$ | H | H | 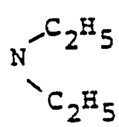 | 70 | 246°C |
| 25 | $(CH_3)_2N$-CO | $C_2H_5$ | $C_6H_5$ | H | H | 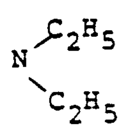 | 66 | 167° |
| 26 | $CH_3NH$-CO | $C_2H_5$ | $C_6H_5$ | H | H | 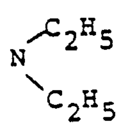 | 43 | 197° |

Tabelle 3 (Fortsetzung)

| Beispiel-Nr. | $R^1$ | $R^2$ | $R^3$ | $R^4$ | $R^5$ | 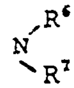 | Ausbeute % | Fp. |
|---|---|---|---|---|---|---|---|---|
| 27 | 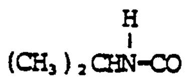 | $C_6H_5$ | $C_6H_5$ | H | H | 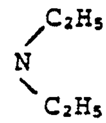 | 84 | 181°C |
| 28 | 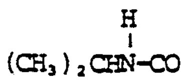 | $CH_3$ | $C_6H_5$ | H | H | 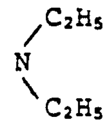 | 81 | 207°C |
| 29 | $C_6H_5$ | $CH_3$ | $C_6H_5$ | H | H | 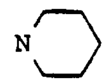 | 78 | 238°C |
| 30 | H | H | $C_6H_5$ | H | H | 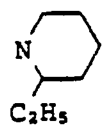 | 70 | 230°C |
| 31 | H | H | $C_6H_5$ | H | H | 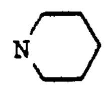 | 75 | 292°C |
| 32 | H | H | $C_6H_5$ | H | H | 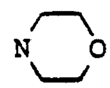 | 70 | 208°C |
| 33 | 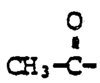 | H | $C_6H_5$ | H | H | 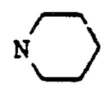 | 90 | 264°C |
| 34 | 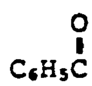 | H | $C_6H_5$ | H | H | 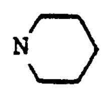 | 80 | 241°C |
| 35 | 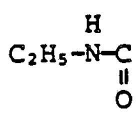 | H | $C_6H_5$ | H | H | 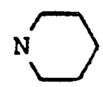 | 90 | 190°C |
| 36 | 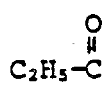 | H | $C_6H_5$ | H | H | 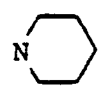 | 85 | 231°C |
| 37 | 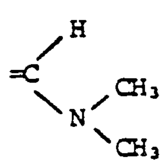 | | $C_6H_5$ | H | H | 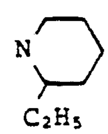 | 90 | 182°C |
| 38 | 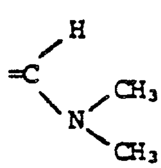 | | $C_6H_5$ | H | H | 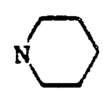 | 92 | 206-8°C |
| 39 | 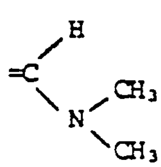 | | $C_6H_5$ | H | H | 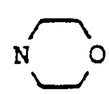 | 90 | 223°C |

Tabelle 3 (Fortsetzung)

| Beispiel-Nr. | $R^1$ $R^2$ | $R^3$ | $R^4$ | $R^5$ | 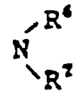 | Ausbeute % | Fp. |
|---|---|---|---|---|---|---|---|
| 40 | 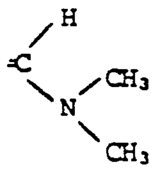 | $C_6H_5$ | H | H | 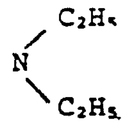 | 96 | 173°C |
| 41 | 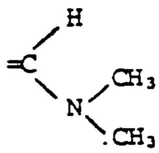 | $C_6H_5$ | H | H | 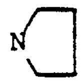 | 90 | 206°C |
| 42 | 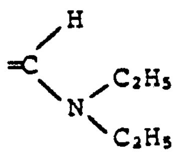 | $C_6H_5$ | H | H | 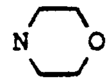 | 80 | 174°C |
| 43 | 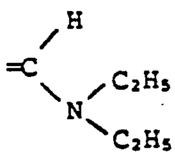 | $C_6H_5$ | H | H | 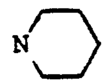 | 90 | 120°C |
| 44 | 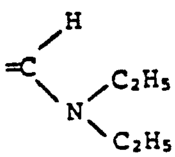 | $C_6H_5$ | H | H | 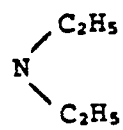 | 85 | 136°C |
| 45 | 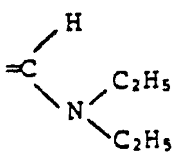 | $C_6H_5$ | H | H | 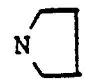 | 90 | 168°C |
| 46 | 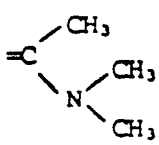 | $C_6H_5$ | H | H | 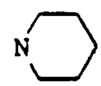 | 70 | 220°C |
| 47 | 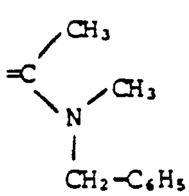 | $C_6H_5$ | H | H | 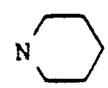 | 60 | 165°C |
| 48 | 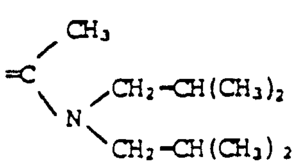 | $C_6H_5$ | H | H | 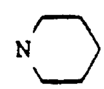 | 70 | 184°C |

**Patentansprüche** (für die Vertragsstaaten : BE, CH, DE, FR, GB, IT, LI, LU, NL, SE)

1. Imidazothiadiazolalkencarbonsäureamide der allgemeinen Formel (I)

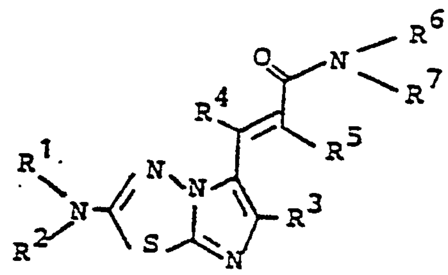

(I)

in welcher

$R^1$ für Wasserstoff, Phenyl oder einen geradkettigen verzweigten oder cyclischen gesättigten oder ungesättigten Kohlenwasserstoffrest mit bis zu 6 Kohlenstoffatomen steht, der gegebenenfalls einmal durch Sauerstoff, NH, N-Alkyl mit 1 bis 4 C-Atomen oder N-Benzyl unterbrochen ist und der gegebenenfalls substituiert ist durch Halogen,

$R^2$ entweder für den Rest $CXR^8$ steht, wobei $R^8$ die für $R^1$ angegebene Bedeutung hat (gleich oder verschieden) und wobei X für Sauerstoff oder den Rest $NR^9R^{10}$ steht, wobei $R^9$ und $R^{10}$ gleich oder verschieden sind und jeweils die für $R^1$ angegebene Bedeutung besitzen oder

$R^1$ und $R^2$ gemeinsam für eine Gruppe der allgemeinen Formel $=C(R^{12})(Y\text{-}R^{13})$ stehen, wobei Y für den Rest N-Alkyl mit 1 bis 4 C-Atomen steht und $R^{12}$ und $R^{13}$ gleich oder verschieden sind und jeweils die für $R^1$ angegebene Bedeutung besitzen und mit $R^1$ gleich oder verschieden sind,

$R^3$ die für $R^1$ angegebene Bedeutung besitzt und mit $R^1$ gleich oder verschieden ist oder für Phenyl, Furyl, Thienyl, Pyrimidyl oder Pyridyl steht, wobei die Ringe gegebenenfalls substituiert sind durch ein oder zwei gleiche oder verschiedene Substituenten aus der Gruppe Halogen, Nitro, Trifluormethyl, Phenyl, Alkyl, Alkoxy, Dialkylamino oder $SO_n$-Alkyl ($n = 0$ oder 2), wobei die genannten Alkyl- oder Alkoxy-Reste 1 bis 4 Kohlenstoffatome enthalten,

$R^4$ und $R^5$ gleich oder verschieden sind und für Wasserstoff oder Alkyl mit 1 bis 4 Kohlenstoffatomen stehen und

$R^6$ und $R^7$ die für $R^1$ angegebene Bedeutung besitzen und mit $R^1$ gleich oder verschieden sind oder gemeinsam mit dem Stickstoffatom einen 5- bis 7-gliedrigen Ring bilden, der gegebenenfalls durch ein Sauerstoff- oder Stickstoffatom unterbrochen ist, wobei der Stickstoff gegebenenfalls durch Wasserstoff, Alkyl mit 1 bis 4 Kohlenstoffatomen oder Benzyl substituiert ist und wobei dieser 5- bis 7-gliedrige Ring gegebenenfalls durch 1 oder 2 gleiche oder verschiedene Substituenten aus der Gruppen Halogen, Benzyl, Trifluormethyl, Alkyl oder Alkoxy mit jeweils 1 bis 4 Kohlenstoffatomen substituiert ist oder wobei dieser Ring gegebenenfalls in einem aromatischen Ring mit 6 oder 10 C-Atomen kondensiert sein kann, sowie ihre stereoisomeren Formen der verschiedenen Enantiomeren, Diastereomeren und Z/E-Isomeren sowie ihre pharmazeutisch unbedenklichen Säureadditionssalze.

2. Verfahren zur Herstellung von Imidazothiadiazolalkencarbonsäureamiden der allgemeinen Formel (I)

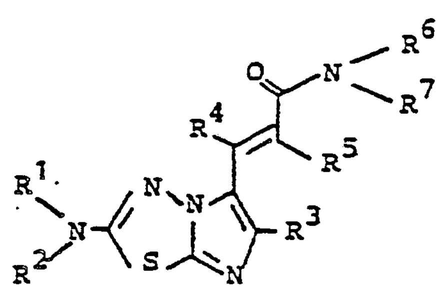

in welcher $R^1$ bis $R^7$ die in Anspruch 1 angegebenen Bedeutungen haben, sowie ihre stereoisomeren Formen der verschiedenen Enantiomeren, Diastereomeren und Z/E-Isomeren sowie ihre pharmazeutisch unbedenklichen Säureadditionssalze, dadurch gekennzeichnet, daß man

a) Carbonylverbindungen der allgemeinen Formel (II)

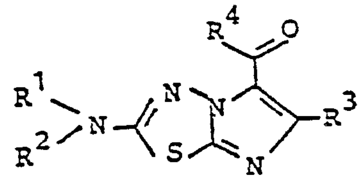

(II)

in welcher $R^1$ bis $R^4$ die oben angegebene Bedeutung haben, mit Phosphonatverbindungen der allgemeinen Formel (III)

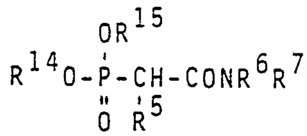

(III)

in welcher

$R^5$ bis $R^7$ die oben angegebene Bedeutung haben, und

$R^{14}$ und $R^{15}$ für gegebenenfalls substituiertes Alkyl oder Aralkyl stehen,

in Gegenwart von starken Basen und in inerten organischen Lösungsmitteln bei Temperaturen zwischen —20 und 110 °C umsetzt, wobei im Falle $R^8 = CH_3$ und X = 0, Alkencarbonsäureamide der allgemeinen Formel (I) mit $R^2$ = H erhalten werden oder

b) Alkencarbonsäureamide der allgemeinen Formel (I), in der $R^1$ bis $R^7$ die oben angegebene Bedeutung haben und $R^2$ gleich Wasserstoff ist, mit geeigneten Acylierungsmitteln der allgemeinen Formel (IV)

$$R^{2'}—Cl \qquad (IV)$$

in welcher $R^{2'}$ die oben für $R^2$ angegebene Bedeutung besitzt, aber nicht Wasserstoff bedeutet, in inerten organischen Lösungsmitteln, gegebenenfalls in Gegenwart einer organischen Base, umsetzt, oder

c) Carbonylverbindungen der allgemeinen Formel (II) mit Acetamidderivaten der allgemeinen Formel (V)

$$R^5—CH_2—CONR^6R^7 \qquad (V)$$

in welcher $R^5$, $R^6$ und $R^7$ die oben angegebene Bedeutung haben, in Gegenwart von sauren oder basischen Katalysatoren und gegebenenfalls in Gegenwart von inerten organischen Lösungsmitteln bei Temperaturen zwischen 20 und 200 °C umsetzt, oder

d) Alkencarbonsäuren der allgemeinen Formel (VI)

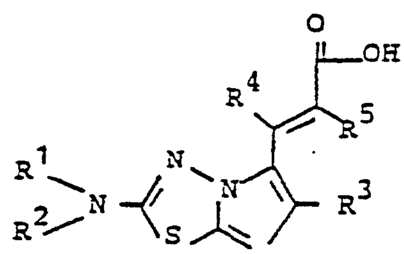

(VI)

in welcher $R^1$ bis $R^5$ die oben angegebene Bedeutung haben, mit Aminen der allgemeinen Formel (VII)

$$HNR^6R^7 \qquad (VII)$$

in welcher $R^6$ und $R^7$ die oben angegebene Bedeutung haben, gegebenenfalls nach Aktivierung der Carboxylgruppe über das entsprechende Säurechlorid z. B. (durch Thionylchlorid) in organischen inerten Lösungsmitteln bei Temperaturen zwischen 20 und 150 °C amidiert, und sie gegebenenfalls in Säureadditionssalze überführt.

3. Verbindungen der allgemeinen Formel (I) gemäß Anspruch 1 zur Verwendung bei der Bekämpfung von Erkrankungen.

4. Arzneimittel, enthaltend mindestens eine Verbindung der allgemeinen Formel (I) gemäß Anspruch 1.

5. Verfahren zur Herstellung von Arzneimitteln dadurch gekennzeichnet, daß man Verbindungen der allgemeinen Formel (I) gemäß Anspruch 1 gegebenenfalls unter Verwendung üblicher Hilfs- und Trägerstoffe in eine geeignete Applikationsform überführt.

6. Verwendung von Verbindungen der allgemeinen Formel (I) gemäß Anspruch 1 zur Herstellung von Arzneimitteln.

7. Carbonylverbindungen der allgemeinen Formel (II)

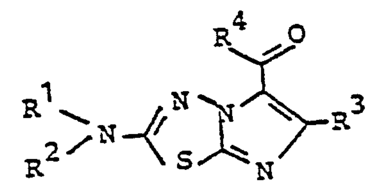

(II)

in welcher $R^1$ bis $R^4$ die in Anspruch 1 angegebene Bedeutung haben.

8. Alkencarbonsäuren der allgemeinen Formel (VI)

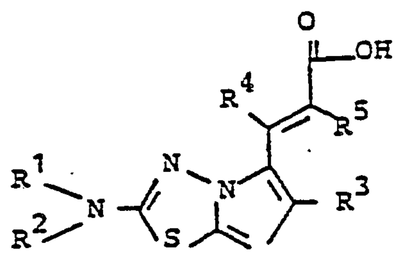

(VI)

in welcher $R^1$ bis $R^5$ die in Anspruch 1 angegebene Bedeutung haben.

**Patentansprüche** (für den Vertragsstaat AT)

1. Verfahren zur Herstellung von Imidazothiadiazolalkencarbonsäureamiden der allgemeinen Formel (I)

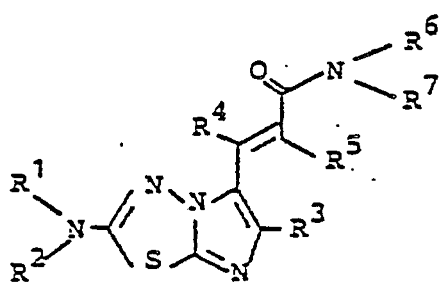

in welcher

$R^1$ für Wasserstoff, Phenyl oder einen geradkettigen verzweigten oder cyclischen gesättigten oder ungesättigten Kohlenwasserstoffrest mit bis zu 6 Kohlenstoffatomen steht, der gegebenenfalls einmal durch Sauerstoff, NH, N-Alkyl mit 1 bis 4 C-Atomen oder N-Benzyl unterbrochen ist und der gegebenenfalls substituiert ist durch Halogen,

$R^2$ entweder für den Rest $CXR^8$ steht, wobei $R^8$ die für $R^1$ angegebene Bedeutung hat (gleich oder verschieden) und wobei X für Sauerstoff oder den Rest $NR^9R^{10}$ steht, wobei $R^9$ und $R^{10}$ gleich oder verschieden sind und jeweils die für $R^1$ angegebene Bedeutung besitzen oder

$R^1$ und $R^2$ gemeinsam für eine Gruppe der allgemeinen Formel $= C(R^{12})(Y\text{-}R^{13})$ stehen, wobei Y für den Rest N-Alkyl mit 1 bis 4 C-Atomen steht und $R^{12}$ und $R^{13}$ gleich oder verschieden sind und jeweils die für $R^1$ angegebene Bedeutung besitzen und mit $R^1$ gleich oder verschieden sind,

$R^3$ die für $R^1$ angegebene Bedeutung besitzt und mit $R^1$ gleich oder verschieden ist oder für Phenyl, Furyl, Thienyl, Pyrimidyl oder Pyridyl steht, wobei die Ringe gegebenenfalls substituiert sind durch ein oder zwei gleiche oder verschiedene Substituenten aus der Gruppe Halogen, Nitro, Trifluormethyl, Phenyl, Alkyl, Alkoxy, Dialkylamino oder $SO_n$-Alkyl (n = 0 oder 2), wobei die genannten Alkyl- oder Alkoxy-Reste 1 bis 4 Kohlenstoffatome enthalten,

$R^4$ und $R^5$ gleich oder verschieden sind und für Wasserstoff oder Alkyl mit 1 bis 4 Kohlenstoffatomen stehen und

$R^6$ und $R^7$ die für $R^1$ angegebene Bedeutung besitzen und mit $R^1$ gleich oder verschieden sind oder gemeinsam mit dem Stickstoffatom einen 5- bis 7-gliedrigen Ring bilden, der gegebenenfalls durch ein Sauerstoff- oder Stickstoffatom unterbrochen ist wobei der Stickstoff gegebenenfalls durch Wasserstoff, Alkyl mit 1 bis 4 Kohlenstoffatomen oder Benzyl substituiert ist und wobei dieser 5- bis 7-gliedrige Ring gegebenenfalls durch 1 oder 2 gleiche oder verschiedene Substituenten aus der Gruppe Halogen, Benzyl, Trifluormethyl, Alkyl oder Alkoxy mit jeweils 1 bis Kohlenstoffatomen substituiert ist oder wobei dieser Ring gegebenenfalls mit einem aromatischen Ring mit 6 oder 10 C-Atomen kondensiert sein kann, sowie ihre stereoisomeren Formen der verschiedenen Enantiomeren, Diastereomeren und Z/E-Isomeren sowie ihre pharmazeutisch unbedenklichen Säureadditionssalze, dadurch gekennzeichnet, daß man

a) Carbonylverbindungen der allgemeinen Formel (II)

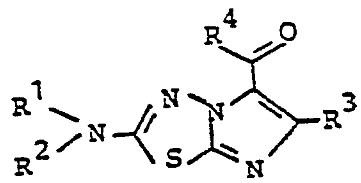

(II)

in welcher $R^1$ bis $R^4$ die oben angegebene Bedeutung haben, mit Phosphonatverbindungen der allgemeinen Formel (III)

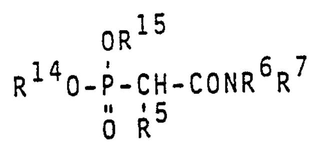

(III)

in welcher

$R^5$ bis $R^7$ die oben angegebene Bedeutung haben, und

$R^{14}$ und $R^{15}$ für gegebenenfalls substituiertes Alkyl oder Aralkyl stehen,

in Gegenwart von starken Basen und in inerten organischen Lösungsmitteln bei Temperaturen zwischen —20 und 110 °C umsetzt, wobei, im Falle $R^8 = CH_3$ und X = 0, Alkencarbonsäureamide der allgemeinen Formel (I) mit $R^2$ = H erhalten werden oder

b) Alkencarbonsäureamide der allgemeinen Formel (I), in der $R^1$ bis $R^7$ die oben angegebene Bedeutung haben und $R^2$ gleich Wasserstoff ist, mit geeigneten Acylierungsmitteln der allgemeinen Formel (IV)

$$R^{2'}—Cl \qquad (IV)$$

in welcher $R^{2'}$ die oben für $R^2$ angegebene Bedeutung besitzt, aber nicht Wasserstoff bedeutet, in inerten organischen Lösungsmitteln, gegebenenfalls in Gegenwart einer organischen Base, umsetzt, oder

c) Carbonylverbindungen der allgemeinen Formel (II) mit Acetamidderivaten der allgemeinen Formel (V)

$$R^5—CH_2—CONR^6R^7 \qquad (V)$$

in welcher $R^5$, $R^6$ und $R^7$ die oben angegebene Bedeutung haben, in Gegenwart von sauren oder basischen Katalysatoren und gegebenenfalls in Gegenwart von inerten organischen Lösungsmitteln bei Temperaturen zwischen 20 und 200 °C umsetzt, oder

d) Alkencarbonsäuren der allgemeinen Formel (VI)

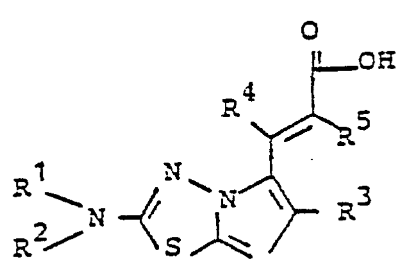

(VI)

in welcher $R^1$ bis $R^5$ die oben angegebene Bedeutung haben, mit Aminen der allgemeinen Formel (VII)

$$HNR^6R^7 \quad (VII)$$

in welcher $R^6$ und $R^7$ die oben angegebene Bedeutung haben, gegebenenfalls nach Aktivierung der Carboxylgruppe über das entsprechende Säurechlorid z. B. (durch Thionylchlorid) in organischen inerten Lösungsmitteln bei Temperaturen zwischen 20 und 150 °C amidiert, und sie gegebenenfalls in Säureadditionssalze überführt.

2. Verfahren zur Herstellung von Arzneimitteln, dadurch gekennzeichnet, daß man Verbindungen der allgemeinen Formel (I) gemäß Anspruch 1, gegebenenfalls unter Verwendung üblicher Hilfs- und Trägerstoffe in eine geeignete Applikationsform überführt.

3. Verwendung von Verbindungen der allgemeinen Formel (I) gemäß Anspruch 1 zur Herstellung von Arzneimitteln.

**Claims** (for the Contracting States : BE, CH, DE, FR, GB, IT, LI, LU, NL, SE)

1. Imidazothiadiazolealkenecarboxylic acid amides of the general formula (I)

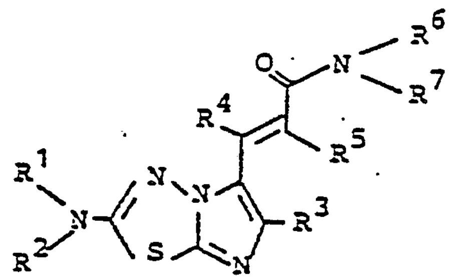

(I)

in which

$R^1$ represents hydrogen, phenyl or a straight-chain, branched or cyclic, saturated or unsaturated hydrocarbon radical with up to 6 carbon atoms, which is optionally interrupted once by oxygen, NH, N-alkyl with 1 to 4 C atoms or N-benzyl and which is optionally substituted by halogen,

$R^2$ either represents the radical $CXR^8$, wherein $R^8$ has the meaning given for $R^1$ (identical or different) and wherein X represents oxygen or the radical $NR^9R^{10}$, wherein $R^9$ and $R^{10}$ are identical or different and each have the meaning given for $R^1$, or

$R^1$ and $R^2$ together represent a group of the general formula $= C(R^{12})(Y\text{-}R^{13})$, wherein Y represents the radical N-alkyl with 1 to 4 C atoms and $R^{12}$ and $R^{13}$ are identical or different and each have the meaning given for $R^1$ and are identical to or different from $R^1$,

$R^3$ has the meaning given for $R^1$ and is identical to or different from $R^1$ or represents phenyl, furyl, thienyl, pyrimidyl or pyridyl, the rings optionally being substituted by one or two identical or different substituents from the group comprising halogen, nitro, trifluoromethyl, phenyl, alkyl, alkoxy, dialkylamino or $SO_n$-alkyl ($n = 0$ or 2), the alkyl or alkoxy radicals mentioned containing 1 to 4 carbon atoms,

$R^4$ and $R^5$ are identical or different and represent hydrogen or alkyl with 1 to 4 carbon atoms and

$R^6$ and $R^7$ have the meaning given for $R^1$ and are identical to or different from $R^1$, or, together with the nitrogen atom, form a 5- to 7-membered ring, which is optionally interrupted by an oxygen or nitrogen atom, the nitrogen optionally being substituted by hydrogen, alkyl with 1 to 4 carbon atoms or benzyl, and this 5- to 7-membered ring optionally being substituted by 1 or 2 identical or different substituents from the group comprising halogen, benzyl, trifluoromethyl, alkyl or alkoxy with in each case 1 to 4 carbon atoms, or it being possible for this ring optionally to be condensed with an aromatic ring with 6 or 10 C atoms,

and their stereoisomeric forms of the various enantiomers, diastereomers and Z/E isomers and their pharmaceutically acceptable acid addition salts.

2. Process for the preparation of imidazothiadiazolealkenecarboxylic acid amides of the general formula (I)

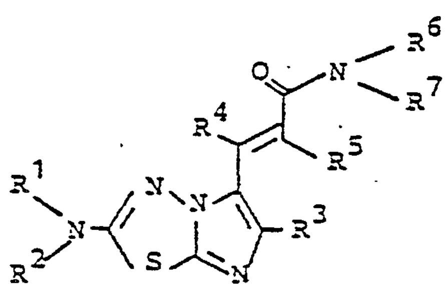

in which $R^1$ to $R^7$ have the meanings given in Claim 1, and their stereoisomeric forms of the various enantiomers, diastereomers and Z/E isomers and their pharmaceutically acceptable acid addition salts, characterised in that

a) carbonyl compounds of the general formula (II)

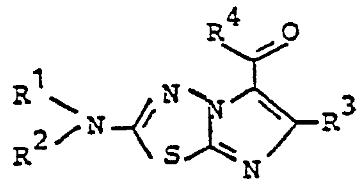

(II)

in which $R^1$ to $R^4$ have the abovementioned meaning, are reacted with phosphonate compounds of the general formula (III)

$$R^{14}O-\underset{\overset{\|}{O}}{\overset{OR^{15}}{P}}-\underset{R^{5}}{CH}-CONR^{6}R^{7} \qquad (III)$$

in which

$R^5$ to $R^7$ have the abovementioned meaning and

$R^{14}$ and $R^{15}$ represent optionally substituted alkyl or aralkyl,

in the presence of strong bases and in inert organic solvents at temperatures of between —20 and 110 °C, alkenecarboxylic acid amides of the general formula (I) in which $R^2 = H$ being obtained in the case where $R^8 = CH_3$ and $X = 0$, or

b) alkenecarboxylic acid amides of the general formula (I), in which $R^1$ to $R^7$ have the abovementioned meaning and $R^2$ is hydrogen, are reacted with suitable acylating agents of the general formula (IV)

$$R^{2'}—Cl \qquad (IV)$$

in which $R^{2'}$ has the meaning given above for $R^2$, but does not denote hydrogen, in inert organic solvents, optionally in the presence of an organic base, or

c) carbonyl compounds of the general formula (II) are reacted with acetamide derivatives of the general formula (V)

$$R^5—CH_2—CONR^6R^7 \qquad (V)$$

in which $R^5$, $R^6$ and $R^7$ have the abovementioned meaning, in the presence of acid or basic catalysts and optionally in the presence of inert organic solvents at temperatures of between 20 and 200 °C, or

d) alkenecarboxylic acids of the general formula (VI)

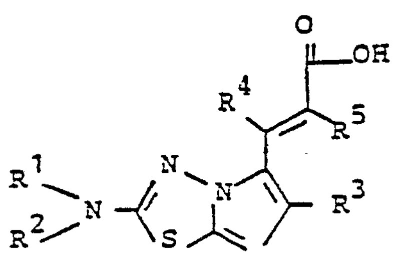

(VI)

in which $R^1$ to $R^5$ have the abovementioned meaning, are amidated with amines of the general formula (VII)

$HNR^6R^7$ (VII)

in which $R^6$ and $R^7$ have the abovementioned meaning, optionally after activation of the carboxyl group via the corresponding acid chloride for example (by thionyl chloride) in organic inert solvents at temperatures of between 20 and 150 °C, and they are optionally converted into acid addition salts.

3. Compounds of the general formula (I) according to Claim 1 for use in combating diseases.

4. Medicaments containing at least one compound of the general formula (I) according to Claim 1.

5. Process for the preparation of medicaments, characerised in that compounds of the general formula (I) according to Claim 1 are converted into a suitable form of administration, optionally using customary auxiliaries and excipients.

6. Use of compounds of the general formula (I) according to Claim 1 for the preparation of medicaments.

7. Carbonyl compounds of the general formula (II)

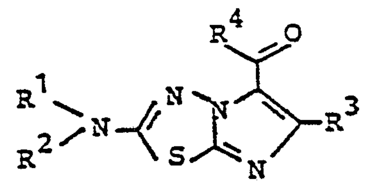

(II)

in which $R^1$ to $R^4$ have the meaning given in Claim 1.

8. Alkenecarboxylic acids of the general formula (VI)

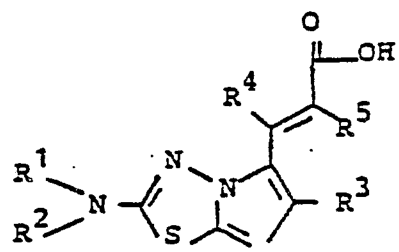

(VI)

in which $R^1$ to $R^5$ have the meaning given in Claim 1.

**Claims** (for the Contracting State AT)

1. Process for the preparation of imidazothiadiazolealkenecarboxylic acid amides of the general formula (I)

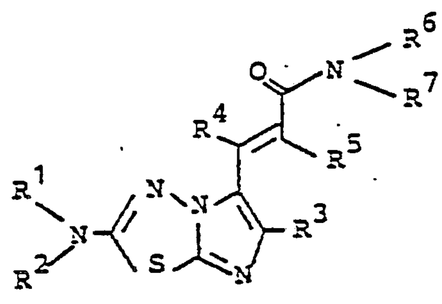

in which

$R^1$ represents hydrogen, phenyl or a straight-chain, branched or cyclic, saturated or unsaturated hydrocarbon radical with 1 to 6 carbon atoms, which is optionally interrupted once by oxygen, NH, N-alkyl with 1 to 4 C atoms or N-benzyl and which is optionally substituted by halogen,

$R^2$ either represents the radical $CXR^8$, wherein $R^8$ has the meaning given for $R^1$ (identical or different) and wherein X represents oxygen or the radical $NR^9R^{10}$, wherein $R^9$ and $R^{10}$ are identical or different and each have the meaning given for $R^1$, or

$R^1$ and $R^2$ together represent a group of the general formula = $C(R^{12})(Y\text{-}R^{13})$, wherein Y represents the radical N-alkyl with 1 to 4 C atoms and $R^{12}$ and $R^{13}$ are identical or different and each have the meaning given for $R^1$ and are identical to or different from $R^1$,

$R^3$ has the meaning given for $R^1$ and is identical to or different from $R^1$ or represents phenyl, furyl, thienyl, pyrimidyl or pyridyl, the rings optionally being substituted by one or two identical or different substituents from the group comprising halogen, nitro, trifluoromethyl, phenyl, alkyl, alkoxy, dialkylamino or $SO_n$-alkyl (n = 0 or 2), the alkyl or alkoxy radicals mentioned containing 1 to 4 carbon atoms,

$R^4$ and $R^5$ are identical or different and represent hydrogen or alkyl with 1 to 4 carbon atoms and

$R^6$ and $R^7$ have the meaning given for $R^1$ and are identical to or different from $R^1$ or, together with the nitrogen atom, form a 5- to 7-membered ring, which is optionally interrupted by an oxygen or nitrogen atom, the nitrogen optionally being substituted by hydrogen, alkyl with 1 to 4 carbon atoms or benzyl, and this 5- to 7-membered ring optionally being substituted by 1 or 2 identical or different substituents from the group comprising halogen, benzyl, trifluoromethyl, alkyl or alkoxy with in each case 1 to carbon atoms or it being possible for this ring optionally to be condensed with an aromatic ring with 6 or 10 C atoms, and their stereoisomeric forms of the various enantiomers, diastereomers and Z/E isomers and their pharmaceutically acceptable acid addition salts, characterised in that

a) carbonyl compounds of the general formula (II)

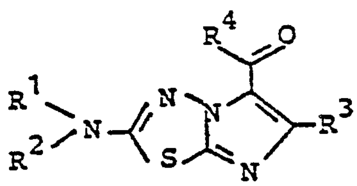

(II)

in which $R^1$ to $R^4$ have the abovementioned meaning, are reacted with phosphonate compounds of the general formula (III)

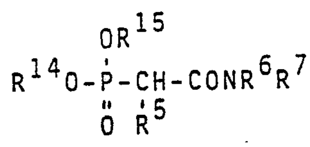

(III)

in which

$R^5$ to $R^7$ have the abovementioned meaning and

$R^{14}$ and $R^{15}$ represent optionally substituted alkyl or aralkyl,

in the presence of strong bases and in inert organic solvents at temperatures of between —20 and 110 °C, alkenecarboxylic acid amides of the general formula (I) in which $R^2$ = H being obtained in the case where $R^8 = CH_3$ and X = 0, or

b) alkenecarboxylic acid amides of the general formula (I), in which $R^1$ to $R^7$ have the abovementioned meaning and $R^2$ is hydrogen, are reacted with suitable acylating agents of the general formula (IV)

$$R^{2'}—Cl \qquad (IV)$$

in which $R^{2'}$ has the meaning given above for $R^2$, but does not denote hydrogen, in inert organic solvents, optionally in the presence of an organic base, or

c) carbonyl compounds of the general formula (II) are reacted with acetamide derivatives of the general formula (V)

$$R^5—CH_2—CONR^6R^7 \qquad (V)$$

in which $R^5$, $R^6$ and $R^7$ have the abovementioned meaning, in the presence of acid or basic catalysts and optionally in the presence if inert organic solvents at temperatures of between 20 and 200 °C, or

d) alkenecarboxylic acids of the general formula (VI)

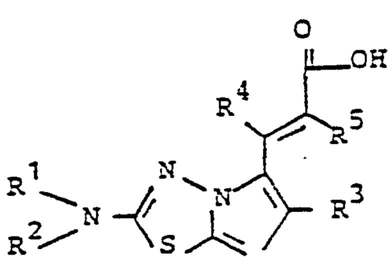

(VI)

in which $R^1$ to $R^5$ have the abovementioned meaning, are amidated with amines of the general formula (VII)

$$HNR^6R^7 \qquad (VII)$$

in which $R^6$ and $R^7$ have the abovementioned meaning, optionally after activation of the carboxyl group via the corresponding acid chloride for example (by thionyl chloride) in organic inert solvents at temperatures of between 20 and 150 °C, and they are optionally converted into acid addition salts.

2. Process for the preparation of medicaments, characterised in that compounds of the general formula (I) according to Claim 1 are converted into a suitable form of administration, optionally using customary auxiliaries and excipients.

3. Use of compounds of the general formula (I) according to Claim 1 for the preparation of medicaments.

**Revendications** (pour les Etats contractants : BE, CH, DE, FR, GB, IT, LI, LU, NL, SE)

1. Amides d'acides imidazothiadiazole-alcène-carboxyliques de formule générale (I)

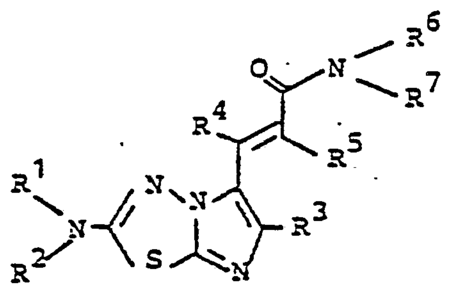

(I)

dans laquelle

$R^1$ désigne l'hydrogène, le groupe phényle ou un reste d'hydrocarbure saturé ou non saturé, à chaîne droite, ramifié ou cyclique, ayant jusqu'à 6 atomes de carbone, qui est interrompu, le cas échéant, une fois par de l'oxygène, un groupe NH, N-alkyle ayant 1 à 4 atomes de carbone ou N-benzyle et qui est éventuellement substitué par un halogène,

$R^2$ désigne le reste $CXR^8$ dans lequel $R^8$ a la définition indiquée pour $R^1$ (identiques ou différents) et X désigne l'oxygène ou le reste $NR^9R^{10}$, $R^9$ et $R^{10}$ étant identiques ou différents et ayant chacun la définition indiquée pour $R^1$ ou bien

$R^1$ et $R^2$ s'associent pour former un groupe de formule générale $= C(R^{12})(Y\text{-}R^{13})$ dans laquelle Y désigne le reste N-alkyle ayant 1 à 4 atomes de carbone et $R^{12}$ et $R^{13}$ sont identiques ou différents et ont chacun la définition indiquée pour $R^1$ et sont identiques à $R^1$ ou en sont différents,

$R^3$ a la définition indiquée pour $R^1$ et est identique à $R^1$ ou en est différent ou représente un groupe phényle, furyle, thiényle, pyrimidyle ou pyridyle, les noyaux étant éventuellement substitués par un ou deux substituants identiques ou différents choisis dans le groupe comprenant un halogène, un reste nitro, trifluorométhyle, phényle, alkyle, alkoxy, dialkylamino ou $SO_n$-alkyle (n = 0 ou 2), les restes alkyle ou alkoxy mentionnés comprenant 1 à 4 atomes de carbone,

$R^4$ et $R^5$ sont identiques ou différents et représentent l'hydrogène ou un groupe alkyle ayant 1 à 4 atomes de carbone et

$R^6$ et $R^7$ ont la définition indiquée pour $R^1$ et sont identiques à $R^1$ ou en sont différents, ou forment conjointement avec l'atome d'azote un noyau pentagonal à heptagonal qui est interrompu le cas échéant par un atome d'oxygène ou d'azote, l'azote étant éventuellement substitué par de l'hydrogène, un reste alkyle ayant 1 à 4 atomes de carbone ou un reste benzyle et ce noyau pentagonal à heptagonal étant éventuellement substitué par un ou deux substituants identiques ou différents choisis dans le groupe comprenant un halogène, un reste benzyle, trifluorométhyle, alkyle ou alkoxy ayant chacun 1 à 4 atomes de carbone, ou bien ce noyau peut éventuellement être condensé en un noyau aromatique ayant 6 ou 10 atomes de carbone,

ainsi que leurs formes stéréo-isomériques des différents énantiomères, diastéréo-isomères et Z/E-isomères, de même que leurs sels d'addition d'acides pharmaceutiquement acceptables.

2. Procédé de production d'amides d'acides imidazothiadiazole-alcène-carboxyliques de formule générale (I)

(Voir formule page suivante)

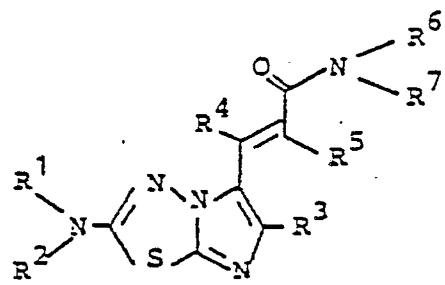

dans laquelle $R^1$ à $R^7$ ont les définitions indiquées dans la revendication 1, ainsi que leurs formes stéréo-isomériques des différents énantiomères, diastéréo-isomères et Z/E-isomères, de même que leurs sels d'addition d'acides pharmaceutiquement acceptables, caractérisé en ce que

a) on fait réagir des composés carbonyliques de formule générale (II)

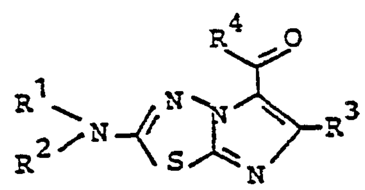

(II)

dans laquelle $R^1$ à $R^4$ ont la définition indiquée ci-dessus, avec des composés du type phosphonate de formule générale (III)

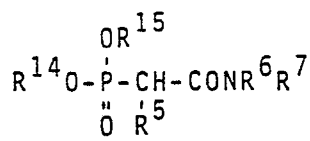

(III)

dans laquelle

$R^5$ à $R^7$ ont la définition indiquée ci-dessus, et

$R^{14}$ et $R^{15}$ représentent un groupe alkyle ou aralkyle éventuellement substitué,

en présence de bases fortes et dans des solvants organiques inertes à des températures comprises entre — 20 et 110 °C, et on obtient alors, pour le cas où $R^8$ est un groupe $CH_3$ et X représente 0, des amides d'acides alcène-carboxyliques de formule générale (I) dans laquelle $R^2$ représente H ou bien

b) on fait réagir des amides d'acides alcène-carboxyliques de formule générale (I) dans laquelle $R^1$ à $R^7$ ont la définition indiquée ci-dessus et $R^2$ représente l'hydrogène, avec des agents acylants appropriés de formule générale (IV)

$$R^{2'}—Cl \qquad (IV)$$

dans laquelle $R^{2'}$ a la définition indiquée ci-dessus pour $R^2$, mais ne représente pas l'hydrogène, dans des solvants organiques inertes, éventuellement en présence d'une base organique, ou bien

c) on fait réagir des composés carbonyliques de formule générale (II) avec des dérivés d'acétamides de formule générale (V)

$$R^5—CH_2—CONR^6R^7 \qquad (V)$$

dans laquelle $R^5$, $R^6$ et $R^7$ ont la définition indiquée ci-dessus, en présence de catalyseurs acides ou basiques et, le cas échéant, en présence de solvants organiques inertes, à des températures comprises entre 20 et 200 °C, ou bien

d) on amide des acides alcène-carboxyliques de formule générale (VI)

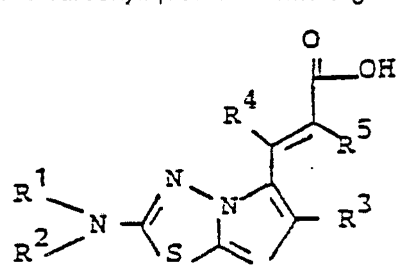

(VI)

dans laquelle $R^1$ à $R^5$ ont la définition indiquée ci-dessus, avec des amines de formule générale (VII)

$$HNR^6R^7 \quad (VII)$$

dans laquelle $R^6$ et $R^7$ ont la définition indiquée ci-dessus, éventuellement après activation du groupe carboxyle en passant par le chlorure d'acide correspondant, par exemple (par le chlorure de thionyle) dans des solvants organiques inertes à des températures comprises entre 20 et 150 °C, et on les transforme éventuellement en sels d'addition d'acides.

3. Composés de formule générale (I) suivant la revendication 1, destinés à être utilisés pour combattre des maladies.

4. Médicaments contenant au moins un composé de formule générale (I) suivant la revendication 1.

5. Procédé de production de médicaments, caractérisé en ce qu'on transforme des composés de formule générale (I) suivant la revendication 1, en une forme d'administration appropriée, en utilisant éventuellement des adjuvants et des excipients classiques.

6. Utilisation de composés de formule générale (I) suivant la revendication 1 pour la préparation de médicaments.

7. Composés carbonyliques de formule générale (II)

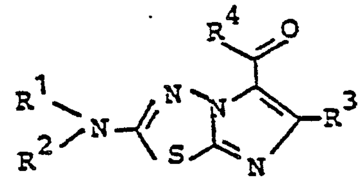

(II)

dans laquelle $R^1$ à $R^4$ ont la définition indiquée dans la revendication 1.

8. Acides alcène-carboxyliques de formule générale (VI)

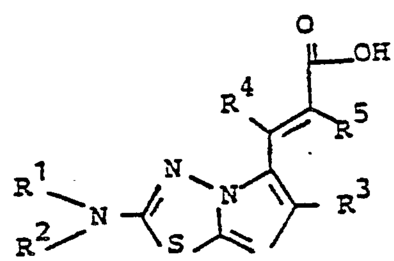

(VI)

dans laquelle $R^1$ à $R^5$ ont la définition indiquée dans la revendication 1.

**Revendications** (pour l'Etat contractant AT)

1. Procédé de production d'amides d'acides imidazothiadiazole-alcène-carboxyliques de formule générale (I)

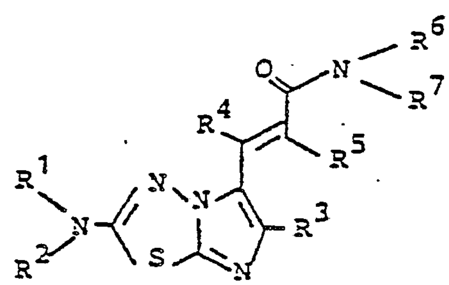

dans laquelle

$R^1$ désigne l'hydrogène, le groupe phényle ou un reste d'hydrocarbure saturé ou non saturé, à chaîne droite, ramifié ou cyclique, ayant jusqu'à 6 atomes de carbone, qui est interrompu, le cas échéant, une fois par de l'oxygène, un groupe NH, N-alkyle ayant 1 à 4 atomes de carbone ou N-benzyle et qui est éventuellement substitué par un halogène.

$R^2$ désigne le reste $CXR^8$ dans lequel $R^8$ a la définition indiquée pour $R^1$ (identiques ou différents) et X désigne l'oxygène ou le reste $NR^9R^{10}$, $R^9$ et $R^{10}$ étant identiques ou différents et ayant chacun la définition indiquée pour $R^1$ ou bien

$R^1$ et $R^2$ s'associent pour former un groupe de formule générale $= C(R^{12})(Y—R^{13})$ dans laquelle Y désigne le reste N-alkyle ayant 1 à 4 atomes de carbone et $R^{12}$ et $R^{13}$ sont identiques ou différents et ont chacun la définition indiquée pour $R^1$ et sont identiques à $R^1$ ou en sont différents.

$R^3$ a la définition indiquée pour $R^1$ et est identique à $R^1$ ou en est différent ou représente un groupe phényle, furyle, thiényle, pyrimidyle ou pyridyle, les noyaux étant éventuellement substitués par un ou deux substituants identiques ou différents choisis dans le groupe comprenant un halogène, un reste nitro, trifluorométhyle, phényle, alkyle, alkoxy, dialkylamino ou $SO_n$-alkyle (n = 0 ou 2), les restes alkyle ou alkoxy mentionnés comprenant 1 à 4 atomes de carbone,

$R^4$ et $R^5$ sont identiques ou différents et représentent l'hydrogène ou un groupe alkyle ayant 1 à 4 atomes de carbone et

$R^6$ et $R^7$ ont la définition indiquée pour $R^1$ et sont identiques à $R^1$ ou en sont différents, ou forment conjointement avec l'atome d'azote un noyau pentagonal à heptagonal qui est interrompu le cas échéant par un atome d'oxygène ou d'azote, l'azote étant éventuellement substitué par de l'hydrogène, un reste alkyle ayant 1 à 4 atomes de carbone ou un reste benzyle et ce noyau pentagonal à heptagonal étant éventuellement substitué par un ou deux substituants identiques ou différents choisis dans le groupe comprenant un halogène, un reste benzyle, trifluorométhyle, alkyle ou alkoxy ayant chacun 1 à 4 atomes de carbone, ou bien ce noyau peut éventuellement être condensé en un noyau aromatique ayant 6 ou 10 atomes de carbone,

ainsi que leurs formes stéréo-isomériques des divers énantiomères, diastéréo-isomères et Z/E-isomères, de même que leurs sels d'addition d'acides pharmaceutiquement acceptables, caractérisé en ce que

a) on fait réagir des composés carbonyliques de formule générale (II)

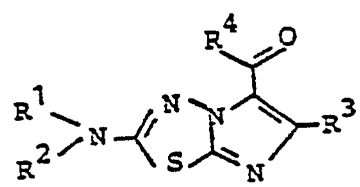

(II)

dans laquelle $R^1$ à $R^4$ ont la définition indiquée ci-dessus, avec des composés du type phosphonate de formule générale (III)

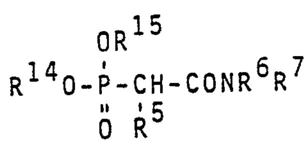

(III)

dans laquelle

$R^5$ à $R^7$ ont la définition indiquée ci-dessus, et

$R^{14}$ et $R^{15}$ représentent un groupe alkyle ou aralkyle éventuellement substitué,

en présence de bases fortes et dans des solvants organiques inertes à des températures comprises entre — 20 et 110 °C, et on obtient alors, pour le cas où $R^8$ est un groupe $CH_3$ et X représente 0, des amides d'acides alcène-carboxyliques de formule générale (I) dans laquelle $R^2$ représente H ou bien

b) on fait réagir des amides d'acides alcène-carboxyliques de formule générale (I) dans laquelle $R^1$ à $R^7$ ont la définition indiquée ci-dessus et $R^2$ représente l'hydrogène, avec des agents acylants appropriés de formule générale (IV)

$$R^{2'}—Cl \qquad (IV)$$

dans laquelle $R^{2'}$ a la définition indiquée ci-dessus pour $R^2$, mais ne représente pas l'hydrogène, dans des solvants organiques inertes, éventuellement en présence d'une base organique, ou bien

c) on fait réagir des composés carbonyliques de formule générale (II) avec des dérivés d'acétamides de formule générale (V)

$$R^5—CH_2—CONR^6R^7 \qquad (V)$$

dans laquelle $R^5$, $R^6$ et $R^7$ ont la définition indiquée ci-dessus, en présence de catalyseurs acides ou basiques et, le cas échéant, en présence de solvants organiques inertes, à des températures comprises entre 20 et 200 °C, ou bien

d) on amide des acides alcène-carboxyliques de formule générale (VI)

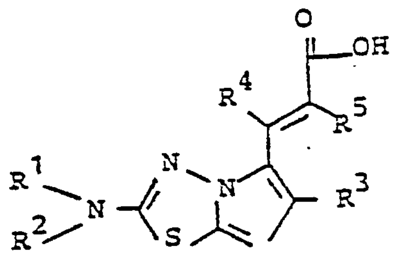

(VI)

dans laquelle $R^1$ à $R^5$ ont la définition indiquée ci-dessus, avec des amines de formule générale (VII)

$$HNR^6R^7 \qquad (VII)$$

dans laquelle $R^6$ et $R^7$ ont la définition indiquée ci-dessus, éventuellement après activation du groupe carboxyle en passant par le chlorure d'acide correspondant, par exemple (par le chlorure de thionyle) dans des solvants organiques inertes à des températures comprises entre 20 et 150 °C, et on les transforme éventuellement en sels d'addition d'acides.

2. Procédé de préparation de médicaments, caractérisé en ce qu'on transforme des composés de formule générale (I) suivant la revendication 1 en une forme d'application appropriée, en utilisant éventuellement des substances auxiliaires et des excipients classiques.

3. Utilisation de composés de formule générale (I) suivant la revendication 1 pour la préparation de médicaments.